Europäisches Patentamt

European Patent Office

Office européen des brevets

(11) Publication number: 0 018 779
A1

## EUROPEAN PATENT APPLICATION

(21) Application number: 80301327.5

(22) Date of filing: 24.04.80

(51) Int. Cl.³: G 01 N 33/50
A 61 K 35/16

(30) Priority: 25.04.79 IL 57135

(43) Date of publication of application:
12.11.80 Bulletin 80/23

(84) Designated Contracting States:
AT BE CH DE FR GB IT LU NL SE

(71) Applicant: Klein, Ami, Dr.

Beit Yitzhak(IL)

(72) Inventor: Klein, Ami, Dr.

Beit Yitzhak(IL)

(74) Representative: Green, Alan James
Sanderson & Co. 97 High Street
Colchester Essex(GB)

(54) A method for the diagnosis of a factor deficiency disease, a test kit for use in carrying out the method and the use of an agent according to the method in the treatment of patients.

(57) The invention relates to a method for the diagnosis of disease, especially cancer, by determining in plasma or serum from a suspected patient the concentration of a factor or factors which enhances the cortisol metabolism in lymphocytes or leucocytes. Thus, the rate at which cortisol is metabolised in lymphocytes or leucocytes in the presence of serum or plasma from a healty donor and the rate at which cortisol is metabolised in lymphocytes or leucocytes in the presence of serum or plasma from a patient under test are determined, and a significantly reduced rate of metabolism is indicative of the presence of disease.

EP 0 018 779 A1

DIAGNOSTIC METHOD

The present invention relates to a diagnostic method for the determination of a factor or factors (hereinafter called for short "LCMEF"), which enhances the cortisol metabolism in lymphocytes or leucocytes (hereinafter called for short "lymphocytes"), in serum or plasma (hereinafter called for short "plasma") and to a test kit to be used for this method. The method is particularly suitable for the diagnosis of cancer.

It is possible to demonstrate in the plasma of patients with a cancerous disease the presence of antigens which are associated with neoplasma or tumors in certain organs. This can be achieved by chemical, and especially immuno-chemical, techniques.

It is known that lymphocytes of cancer patients have an enhanced cortisol metabolism in comparison with that of healthy donors, whereby cortisol is metabolised to e.g. tetrahydro-cortisol, $20\alpha$-hydrocortisol and $20\beta$-hydrocortisol. (See Klein et al., Metabolism, Vol. 27, No. 6 (June) 1978). For said metabolism of cortisol a higher activity is found for lymphocytes in a homologous plasma, i.e. plasma and lymphocytes obtained from the same healthy donor, than in a tissue culture medium (RMPI) (Rosewell Park Memorial Institute nutrient). A heterologous plasma, i.e. plasma and lymphocytes not obtained from the same healthy donor, shows a similar activity to that of the homologous plasma.

It should thus have been expected that the plasma of cancer patients would enhance the cortisol metabolism in at least a similar manner to the enhancement generated by the plasma of healthy donors.

It has now been found surprisingly that the enhancement caused by the plasma of cancer patients on the contrary is less than that caused by the plasma of healthy persons. This is apparently due to the fact that in the plasma of cancer patients one or more factors, i.e. LCMEF is either absent or present in a diminished concentration as compared with that found in healthy plasma donors. LCMEF is at least partially responsible for the enhancement of the cortisol metabolism.

It has thus been found that lymphocytes of a healthy donor in a system with a heterologous plasma of a cancer patient show a considerably lower enhancement of the cortisol metabolism than those lymphocytes in a system with a homologous plasma or a heterologous plasma of a healthy donor. Moreover, the removal of their tumors did not lead to an enhanced cortisol metabolism in the plasma of cancer patients.

Accordingly, the present invention provides a method for the diagnosis of a factor deficiency disease, especially cancer, in a suspected patient, which method comprises determining in plasma or serum from the patient the concentration of a factor or factors which enhances the cortisol metabolism in lymphocytes or leucocytes and comparing it with the concentration of said factor or factors in plasma or serum of a healthy donor.

The present invention will be illustrated herein with reference to the diagnosis of cancer. However, it is not restricted thereto and may be utilised for the diagnosis of any disease or malfunction in which the concentration of LCMEF is different in the plasma of healthy persons from that of diseased persons, e.g., people suffering from liver malfunction.

In connection with this invention "lymphocytes of a healthy donor" means lymphocytes and leucocytes of human beings or of other animals. Particularly suitable are the lymphocytes of human beings and of cattle, sheep and swine.

The quantitative determination of the concentration

- 3 -

of LCMEF can be achieved using many common physical, chemical and biological methods. Thus, it can be achieved by the determination of the level of cortisol (labelled or unlabelled) or of one or more of its metabolites. This can be done, for example, by the use of radio-chemical techniques in which lymphocytes, a hydrogen donor agent, the plasma to be tested and a cortisol substrate are incubated. The substrate and metabolites are then extracted, separated and the radioactivity is scanned. If desired, controls using on the one hand a healthy plasma and on the other hand plasma of a known cancer patient can be determined in parallel.

However, it is advantageous to make use of the immuno-chemical properties of LCMEF. For instance, this can be achieved using an immuno precipitation technique without label, such as immuno diffusion, immuno electrophoresis, radial immuno diffusion, rocket electrophoresis and immune electron microscopy, or with label, such as haemagglutination, complement fixation, immuno-fluorescence, radio immuno assay and enzyme immuno assay. Furthermore, a metal can be used as label in an immuno chemical reaction.

Immuno chemical components can be obtained in known manner by immunisation of a proper animal.

The diagnostic determination can be carried out as homogenous heterogenous enzyme immuno assay. Preferably, the immuno chemical determination is carried out as a competitive test, the factor or factors being directly coupled to an enzyme.

In one preferred embodiment of the present invention there is provided a method for diagnosing cancer in a patient comprising the steps of:

(a) preparing a suitable medium containing a hydrogen generating system and a mixture of:

(i) plasma or serum from the patient,

(ii) lymphocytes from a donor known to be free

- 4 -

of cancer (as herein defined), and

(iii)   cortisol;

(b)   incubating said medium for a predetermined period
of time to effect metabolism of the cortisol by
the lymphocytes;

(c)   measuring the rate of metabolism of the cortisol;
and

(d)   comparing said measured rate of metabolism with
the rate of metabolism of cortisol in a reference
medium prepared and incubated in the same manner
as steps (a) and (b) above except that the included
plasma or serum is from a donor known to be free of
cancer.

The hydrogen generating system is preferably a
NADPH generating system.

In a similar manner the above preferred method can
be utilised for the diagnosis of any other disease or
malfunction in which the concentration of LCMEF is
different in the plasma of healthy persons from that of
diseased persons, e.g. people suffering from liver malfunction.

In the case where on incubation of normal lymphocytes with plasma of a healthy donor the resulting
cortisol metabolism rate is regarded as 100%, a comparable rate of 65% metabolism or below for a suspected
patient would be considered as an indication of disease
such as cancer or liver malfunction.

Said method has to be performed substantially at
the physiological pH namely 7.4.  It can be performed
if desired with lyophilised sonicated lymphocytes in a
reconstituted medium.

As will be shown hereinafter the factor or factors
have a molecular weight between 1000 and 10000D.  Moreover, the activity of LCMEF is eliminated by heating the
plasma or a fraction comprising the LCMEF to approximately

- 5 -

$100^{\circ}C$; at pH 1; and under the influence of Pronase.

The addition of plasma comprising LCMEF or preferably the addition of isolated or purified LCMEF to the plasma of cancer patients enhances the cortisol metabolism of the lymphocytes in said patients. Thus the administration of LCMEF can serve as therapy in LCMEF deficient patients and this use of LCMEF is also within the scope of the present invention.

The invention also provides a test kit using radio-chemical techniques including immuno-chemical techniques. A suitable embodiment thereof contains for instance antibodies, optionally insolubilised, the enzyme labelled factor or factors and a substrate that can be converted by the enzyme labelled factor or factors. However, many other combinations are possible.

It is an important advantage of the diagnostic determination and test kit according to the invention, compared with known diagnostic methods, which are based on antigens associated with neoplasma, that the rheuma factor does not interfere.

The present invention will now be illustrated by the following examples without being limited by them.

Example 1

Lymphocytes were prepared from so called "buffy coats" of the blood bank. These were isolated by centrifugation at .400 g using the Ficol Isopac method, washed twice and resuspended in a phosphate buffered saline solution (PBS) with a pH of approximately 7.4 containing penicillin and streptomycin, 100 IU/ml each. This suspension contained $10^8$ lymphocytes per ml and was divided into series of flasks. To each flask a NADPH generating system comprising a solution of 1.0 µmole NADPH, 5.0 µmoles of glucose-6-phosphate, 1.0 Korenberg unit of glucose-6-phosphate dehydrogenase and 1.4 µmoles

- 6 -

of $MgCl_2$ in 0.1 ml PBS, 50 µg of unlabelled cortisol and 5.0 µCi of 1.2-$^3$H-cortisol were added.

To each flask 1.0 ml of one of the following solutions or suspensions was subsequently added:

1) PBS
2) homologous plasma, belonging to the donor of the lymphocytes (HP)
3) heterologous plasma, belonging to another healthy donor (HeP)
4) plasma of a patient with a cancerous disease, which disease had not yet been treated (CPP)
5) HP, that has been heated to approximately 100°C
6) CPP, that has been heated to approximately 100°C
7) heterologous plasma of patients, more than 70 years old, with a non-cancerous disease (HePPa)
8) mixed plasma, consisting of concentrated HP diluted with CPP to the original volume of HP.

A blank contained all components except the lymphocytes.

The sealed flasks were incubated in a shaking bath at 37°C for 17 hours. At the end of the incubation period the contents of each flask were extracted twice with 5 ml of chloroform and evaporated to dryness under a nitrogen atmosphere. The residue was dissolved in ethanol and applied on silica gel HF-254 thin layer plates. The plates were developed in chloroform/methanol (90/10 v/v). Following chromatography the plates were reviewed in UV-light at 254 nm and scanned using a radioactivity scanner (Berthold LB 2723). The product and substrate spots as well as the remainder of the plate were scraped off and extracted with ethanol. The volume of the extract was adjusted to 5.0 ml with ethanol. Samples of 0.1 ml were transferred into scintillation vials and the radioactivity read in a liquid scintillation spectrometer (Packard 3390). The cortisol metabolism was calculated from the data obtained by this procedure.

The results are given in Table I:

TABLE I

| Plasma | Number of measurements | Cortisol conversion** (%) |
|--------|------------------------|---------------------------|
| PBS | 40 | 37 |
| HP | 39 | 100 |
| HeP | 22 | 91 |
| CPP | 36 | 62 |
| HePPa | 5 | 91 |
| HP* | 9 | 63 |
| CPP* | 9 | 57 |
| HP + CPP | 5 | 96 |

&ast; Plasma, that has been heated to approximately 100°C.

&ast;&ast; The conversion of cortisol in HP is set to 100%.

It appears from the data described in the table that CPP shows a considerably lower cortisol metabolism than HP, HeP and HePPa and is of the same order or magnitude as heated HP. In this test and others - carrying out double blind test studies on a sample of patients suffering from various diseases including cancer - a cortisol conversion rate of less than 65% of normal plasma for not heated plasma indicated that the donor has a cancerous disease. It is confirmed by the result for HP + CPP that this decreased metabolism is not due to an inhibition factor in CPP. This shows that the administration of LCMEF can serve as replacement therapy to LCMEF deficient persons.

Example 2

A similar test as described in Example 1 was performed. However, said test was performed with unlabelled

- 8 -

cortisol.  After incubation the percentages of cortisol left were measured in each flask with RIA. (Radio Immuno Assays).

The results obtained are given in Table II.

TABLE II

| Plasma | Non-converted cortisol % |
|--------|--------------------------|
| PBS    | 95%                      |
| HP     | 83%                      |
| CPP    | 92%                      |

The above shows a conversion of 47% for CPP when the HP conversion is taken as 100%.

Example 3

Lymphocytes prepared from so called "buffy coats" of the blood bank were sonicated with the addition of PBS at pH 7.4 in the course of 1 minute.  The mixture obtained was lyophilised and the powder obtained was kept at -20°C.  When the test had to be performed said powder was dissolved in distilled water up to the original cell suspension volume.  Thereafter the test was performed in the same manner as described in Example 1. The results obtained are given in Table III.

TABLE III

| Plasma | Cortisol Conversion % |
|--------|-----------------------|
| PBS    | 58.46%                |
| HP     | 100%                  |
| CPP    | 34.9%                 |

Example 4

A similar test as described in Example 1 was performed. However, the lymphocytes were replaced by leucocytes obtained from a cow. The results obtained are given in Table IV.

TABLE IV

| Plasma | Cortisol Conversion % |
|--------|----------------------|
| PBS | 27% |
| Cow HP | 185% |
| HeP | 100% |
| CPP | 54% |

Example 5

Human plasma was diluted with PBS 1:1. It was ultra-filtrated on an Amicon U.M. 10 membrane. It was found that the factor or factors were in the filtrate which indicated that it had a molecular weight lower than 10,000 D.

Said filtrate was then filtered through an Amicon UM2 membrane and the active fraction was retained indicating that the molecular weight of the factor or factors was higher than 1000 D.

The filtrate obtained after the first filtration was then divided and each portion was subjected to one of the following treatments:

1. heated to $100^{\circ}C$;
2. pH reduced to 1;
3. treated with Pronase of various origins.

One portion was kept untreated.

The cortisol conversion of the treated portions in comparison with the untreated portion is shown in Table V

- 10 -

TABLE V

| Treatment | Cortisol Conversion % |
|---|---|
| PBS | 41.1% |
| Untreated | 100% |
| pH change | 51.5% |
| Heat treatment | 39.2% |
| Pronase | 53.6% |

Example 6

A group of 59 hospital patients were tested as explained in Example 1 for the rate of cortisol conversion. Out of this group 19 were known cancer cases (3 glioma, 1 hepatoma, 2 bladder carcinoma, 2 colon carcinoma, 2 pancreas carcinoma, 5 lung carcinoma, 1 stomach carcinoma, 1 ovary carcinoma, 1 adrenal carcinoma with metastases, 1 hypernephroma). Seventeen out of the nineteen cases had rates below 65% ranging between 28 and 63%. Two cases (1 lung and 1 colon) had values of 87 and 92% respectively and thus in about 10% of the cases false negative results were obtained. Out of the 40 patients with various other diseases, 35 had a rate of above 65% (ranging between 69 and 109%) while 5 cases were below 65% (range 36-64%). These cases were suffering from hepatitis (36% of HP), pneumonia, urinary tract infection, heart disease and Cushing syndrome. Thus in 12.5% of these cases false positive results were obtained.

However, we have additional indications that non-malignant liver malfunctions (including the above hepatitis) almost always (22/23) have a low LCMEF.

Thus the rate of LCMEF indicates also the cases of liver malfunctions. However, in the non-malignant cases the values return to normal in cured patients while they stay low in the malignant cases.

- 11 -

CLAIMS

1.    A method for the diagnosis of a factor deficiency disease in a suspected patient, which method comprises determining in plasma or serum from the patient the concentration of a factor or factors which enhances the cortisol metabolism in lymphocytes or leucocytes and comparing it with the concentration of said factor or factors in plasma or serum of a healthy donor.

2.    A method according to claim 1 for the diagnosis of cancer.

3.    A method according to claim 1 for the diagnosis of liver malfunction.

4.    A method according to any one of the preceding claims, wherein the determination is carried out by a direct method.

5.    A method according to any one of claims 1 to 3, comprising the measurement of cortisol conversion by lymphocytes or leucocytes in the presence of plasma or serum from the suspected patient and from a healthy donor.

6.    A method according to claim 5, comprising radio-chemical measurement of the conversion rate of tritium labelled cortisol.

7.    A method according to claim 5, comprising the measurement by radio imunoassay of the conversion rate of unlabelled cortisol using a radiolabelled cortisol metabolite.

8.    A method according to any one of claims 1 to 3, comprising the determination of the factor or factors by an immunochemical technique.

9.    A method according to any one of the preceding claims, comprising the use of a homogenous competitive test.

10.   A method according to any one of claims 1 to 8, comprising the use of a heterogenous competitive test.

11.   A method for the diagnosis of cancer in a patient comprising the steps of:

(a) preparing a suitable medium including a hydrogen generating system and a mixture of:

    (i) plasma or serum from the patient,

    (ii) lymphocytes from a donor known to be free of cancer (as herein defined), and

    (iii) cortisol;

(b) incubating said medium for a predetermined period of time to effect metabolism of the cortisol by the lymphocytes;

(c) measuring the rate of metabolism of the cortisol; and

(d) comparing said measured rate of metabolism with the rate of metabolism of cortisol in a reference medium prepared and incubated in the same manner as steps (a) and (b) above except that the included plasma or serum is from a donor known to be free of cancer.

12. A method according to claim 11, in which a metabolism of 65% or less than that in the reference medium indicates a cortisol metabolism malfunction linked to a cancer disease.

13. A method according to claim 11 or claim 12, wherein the hydrogen generating system is NADPH.

14. A method according to any one of the preceding claims, wherein the cortisol metabolism rate measurement is performed using regenerated sonicated lymphocytes.

15. A method according to any one of the preceding claims, wherein the cortisol metabolism rate measurement is performed using lymphocytes obtained from human beings.

16. A method according to any one of claims 1 to 14, wherein the cortisol metabolism rate measurement is performed using lymphocytes obtained from non-human animals.

17. A method according to any one of claims 11 to 16, when used for the diagnosis of liver malfunction.

18. A method for the diagnosis of cancer or of liver malfunction substantially as hereinbefore described with reference to any one of Examples 1 to 4.

19. A test kit for use in carrying out a method according to claim 1 and containing: a) antibodies against an immuno compound containing or consisting of the factor or factors, which activate the cortisol metabolism of lymphocytes and are present in a lower concentration or are lacking in blood of patients with a cancerous or like factor deficiency disease, and b) the enzyme labelled factor or factors.

20. A test kit according to claim 19, containing antibodies coupled to an insoluble carrier.

21. A test kit for carrying out a method according to claim 1 or claim 10 and having contained therein at least two of (a) lyophilised sonicated lymphocytes or leucocytes, (b) a hydrogen generating system, and (c) labelled and/or unlabelled cortisol.

22. A method according to claim 1 comprising the use of a test kit according to any one of claims 19 to 21.

23. The use of an agent comprising a factor or factors which enhances the cortisol metabolism in lymphocytes or leucocytes in the treatment of patients displaying low levels of said factor or factors.

## PARTIAL EUROPEAN SEARCH REPORT

which under Rule 45 of the European Patent Convention shall be considered, for the purposes of subsequent proceedings, as the European search report

European Patent Office

Application number

EP 80 30 1327

| Category | Citation of document with indication, where appropriate. of relevant passages | Relevant to claim | CLASSIFICATION OF THE APPLICATION (Int. Cl.³) |
|---|---|---|---|
| **DOCUMENTS CONSIDERED TO BE RELEVANT** | | | |
| X | CHEMICAL ABSTRACTS, vol. 90, no.13, 26th March, 1979, page 355, abstract 101060h. Columbus, Ohio, USA KLEIN et al. "The influence of homologous plasma and fetal calf serum on human lymphocytic cortisol metabolism".<br><br>& Experientia 1979, 35(1), 114-15.<br><br>  * The whole abstract * | 1 | G 01 N 33/50<br>A 61 K 35/16 |
| | -- | | |
| D | CHEMICAL ABSTRACTS, vol. 89, no. 1, 3rd July, 1978, page 386, abstract 4260v. Columbus, Ohio, USA KLEIN et al. "Cortisol metabolism in lymphocytes from cancer-bearing patients".<br><br>& Metab. Clin. Exp. 1978, 27(6), 731-6.<br><br>  * The whole abstract * | 1,2 | **TECHNICAL FIELDS SEARCHED (Int.Cl.³)**<br><br>G 01 N 33/54<br>33/56<br>33/74<br>33/50 |
| | -- | | |
| | CHEMICAL ABSTRACTS, vol. 86, no.25, 20th June, 1977, page 343 | 1,3 | |

-/-

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely:

Claims not searched: 23

Reason for the limitation of the search: Method for treatment of the human or animal body by surgery or therapy (See Art. 52(4) of the European Patent Convention)

**CATEGORY OF CITED DOCUMENTS**

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

| Place of search | Date of completion of the search | Examiner |
|---|---|---|
| The Hague | 14-07-1980 | DE LUCA |

EPO Form 1505.1  06.78

| Category | Citation of document with indication, where appropriate, of relevant passages | Relevant to claim | CLASSIFICATION OF THE APPLICATION (Int Cl ³) |
|---|---|---|---|
| | abstract 186655w. Columbus, Ohio, USA CHAVARRI, M. et al "The effects of temperature and plasma cortisol on distribution of aldosterone between plasma and red blood cells: influence on metabolic clearance rate and on hepatic and renal extraction of aldosterone"<br><br>& J. Clin. Endocrinol. Metab. 1977, 44(4), 752-9.<br><br>    * The whole abstract *<br><br>    -- | | |
| A | CHEMICAL ABSTRACTS, vol. 75, no.3, 19th July,1971, page 62, abstract 15562g. Columbus, Ohio, USA AMARAL,L. et al. "Transport of cortisol by cultured chronic lymphocytic leukemic lymphocytes".<br><br>&  Experientia,1971, 27(5), 511-12.<br><br>    * The whole abstract *<br><br>    -- | 1 | **TECHNICAL FIELDS SEARCHED (Int. Cl.³)** |
| A | FRESENIUS' ZEITSCHRIFT FUR ANA- LYTISCHE CHEMIE, vol. 265, no. 2, 14th June, 1973. G.E. ABRAHAM et al. "Radioimmuno- assay of Plasma Cortisol", page 169.<br><br>    * Abstract *<br><br>    -- | 6 | |
| A | STEROIDS, vol. 32, no. 1, July/ August 1978. KOBAYASHI et al. "Enzyme immuno- assay for cortisol in serum using cortisol 21-amine", pages 137-144.<br><br>    * Page 137, abstract *<br>                    ./.<br><br>    -- | 7-10 | |

European Patent Office

**PARTIAL EUROPEAN SEARCH REPORT**

| | DOCUMENTS CONSIDERED TO BE RELEVANT | | CLASSIFICATION OF THE APPLICATION (Int Cl ³) |
|---|---|---|---|
| Category | Citation of document with indication, where appropriate, of relevant passages | Relevant to claim | |
| | US - A - 3 988 115 (MODABBER) <br> * Abstract; column 1, lines 41-68; column 2, lines 1-68; column 3, lines 1-68; column 4, lines 1-40 * <br> -- | 1,8-10 | |
| AP | FR - A - 2 419 519 (INSTITUT NATIONAL DE LA SANTE ET DE LA RECHERCHE MEDICALE) <br> * Page 2, lines 4-30 * <br> ---- | 11,13 | TECHNICAL FIELDS SEARCHED (Int. Cl.³) |

EPO Form 1505.3  06.78